# EUROPEAN PATENT SPECIFICATION

(11) **EP 0 498 471 B1**
(45) Date of publication and mention of the grant of the patent: **14.08.1996**
(21) Application number: 92106331.9
(22) Date of filing: 13.01.1987
(51) Int. Cl.: A61K 31/71, A61K 9/127, C07H 15/226

(54) **Liposomes comprising a guanidino aminoglycoside**
Guanidinaminoglycosid enthaltende Liposome
Liposomes contenant un guanidino-aminoglycoside

(30) Priority: 23.12.1986 US 946391; 23.12.1986 US 946398
(43) Date of publication of application: 12.08.1992
(62) Divisional of application: 87901176.5
(73) Proprietor: THE LIPOSOME COMPANY, INC., Princeton, NJ 08540 (US)
(72) Inventor: Bally, Marcel B., Vancouver V6T1M8 (CA); Bolcsak, Lois E., Lawrenville NJ 08648 (US); Cullis Pieter R., Vancouver V6J3R2 (CA); Janoff. Andrew S., Yardly PA 19067 (US); Mayer, Lawrence D., Vancouver V6K2A5 (CA); Lenk, Robert P., Lambertville NJ 08530 (US); Jedrusiak, Jo Ann, Robbinsville, NJ 08691 (US)
(74) Representative: Warcoin, Jacques

(56) References cited:
- CHEMICAL ABSTRACTS, vol. 101, no. 2, 9 July 1984, Columbus, Ohio, US; abstract no. 12083w, page 294 ;
- DATABASE WPIL Derwent Publications Ltd., London, GB; AN 85-133430
- DATABASE WPIL Derwent Publications Ltd., London, GB; AN 85-014687

## Description

### FIELD OF THE INVENTION

This invention relates to aminoglycosides, analogs and derivatives thereof, in the form of phosphate and other salts as well as the process for making and utilizing same. Aminoglycoside phosphate liposomes, their preparation and use, are particularly described. Guanidino aminoglycoside liposomes with drug to lipid ratios (w/w) of greater than about 9:25 (eq. wt.) are also disclosed.

### BACKGROUND OF THE INVENTION

Aminoglycosides are a class of compounds characterized by the ability to interfere with protein synthesis in micro-organisms. Aminoglycosides consist of two or more amino sugars joined in a glycoside linkage to a hexose (or aminocyclitol) nucleus. The hexose nuclei thus far known are either streptidine or 2-deoxystreptamine, though others may be anticipated. Aminoglycoside families are distinguished by the amino sugar attached to the aminocyclitol. For example, the neomycin family comprises three amino sugars attached to the central 2-deoxystreptamine. The kanamycin and glutamicin families have only two amino sugars attached to the aminocyclitol.

Aminoglycosides include: neomycin B, paromomycin, ribostamycin, lividomycin, kanamycin A,, kanamycin B, amikacin, tobramycin, viomycin, gentamicin C₁, gentamicin C₁ₐ, (gentamicin C₂, C₁, C₁ₐ and analogs and derivatives thereof collectively "gentamicin"), sisomicin, netilmicin, streptomycin and dihydrostreptomycin. Streptomycin and dihydrostreptomycin characterized by the presence of a guanidino group are understood to be unique in associating with liposomes in higher drug to lipid ratios than the nonguanidino aminoglycosides. The term "nonguanidino" aminoglycosides will include aminoglycosides other than aminoglycosides bearing a guanadino group.

Unfortunately, use of these compounds has been limited by several factors. Often directed to use in preventing protein synthesis in bacteria, bacteria have demonstrated a remarkable capacity to resist the inhibitory effect of aminoglycosides. Resistance of an organism to aminoglycoside action occurs with a broad range of aminoglycosides. A further problem of aminoglycoside use has been characteristically poor gastric absorption and rapid excretion. Injection of aminoglycosides results in rapid peak plasma concentration often in the neighborhood of 30 to 90 minutes following intramuscular injection which is associated with toxicity. Another limitation is that the aminoglycosides fail to enter the CNS or the eye.

In the therapeutic use of aminoglycosides in animals, including humans, serious problems of toxicity have been noted. For example, therapeutic use in higher animals may be accompanied by ototoxicity potentially involving both auditory and vestioular functions as well as nephrotoxicity, and neuromuscular blockade culminating in respiratory distress.

CHEMICAL ABSTRACTS, vol. 101, n° 2, 9 July 1984, Columbus Ohio, US ; abstract n° 12093w, p. 294 and DATABASE WPIL, Derwent Publication Ltd., London, GB ; AN 85-133430 disclose streptomycin containing liposomes with a drug : lipid ratio of up to about 3 : 30.

It is an object of this invention to provide an aminoglycoside in the form of a phosphate salt. It is another object of this invention to provide for aminoglycosides with improved liposomal association. It is a further object of this invention to provide a method of manufacture of liposomes associated with aminoglycoside including phosphate salts thereof. It is another object of this invention that said liposomes substantially associate with said aminoglycoside. It is an additional object of this invention that the liposomes of this invention provide a high aminoglycoside to lipid ratio particularly as to nonguanadino aminoglysocides. It is a further object of this invention to provide such liposomes in a pharmaceutical dosage form for therapeutic treatment of an animal including a human.

It is another object of this invention to provide an intravenously administrable form of aminoglycoside without concommitant immediate availability of unbound or unassociated aminoglycoside at high plasma levels.

### SUMMARY OF THE INVENTION

It has now been discovered that aminoglycosides, analogs and derivatives thereof, in the form of phosphate salts and sulfate and other salts, have surprisingly useful therapeutic properties. Aminoglycoside salts are found to be particuarly adapted to association with liposomes. The phosphate salts of aminoglycosides further may have reduced acute toxicity. The term aminoglycoside will be understood to include analogs and derivatives thereof.

The use of aminoglycoside as a phosphate in making liposomes with enhanced loading efficiency is herein disclosed. This is true for guanidino aminoglycosides. The term "associated" shall be understood to be immobilized on or in a liposome, within the aqueous phase of a liposome or within the lipid phase of a liposome.

Weights of aminoglycoside may be expressed as actual weight of the aminoglycoside (actual weight "act.wt.") or the equivalent weight or base equivalent of the drug molecule not including the weight of the counterion (equivalent weight "eq. wt."). The process of this invention permits the preparation of aminoglycoside liposomes (including streptomycin or dihydrostreptomycin) and preparations containing such liposomes to be manufactured in a wide range of potencies higher than obtained without this process. (Guanadino aminoglycoside liposomes by this process attain drug to lipid equivalent weight ratios of greater than about 9:25 (w/w); for example, with greater than about 60 mg streptomycin sulfate/100 mg EPC (act. wt.)).

It is a particular advantage of this invention that enhanced drug to lipid ratio preparations require reduced administration of lipid per drug dosage thus avoiding or reducing toxicity associated with lipid administration.

The enhanced asociation of available drug with liposomes in the preparation of the liposomes reduces the need for drug and lipid starting materials.

Finally, high potency pharmaceutical preparations are consequently of smaller volume and thus cause less tissue insult upon administration. This is particularly true as to intramuscular administration.

Methods of preparing and utilizing aminoglycoside phosphate and aminoglycoside phosphate liposomes are described more fully below.

Methods of preparing and utilizing aminoglycoside liposomes by the modified SPLV process of, in the most preferred embodiment, requiring both drying liposomes to powder and stabilizing liposomes, are also described more fully below.

This invention includes liposomes comprising at least one lipid and at least one phosphate salt of an aminoglycoside. This further includes unilamellar and multilamellar vesicles associated with aminoglycosides phosphates such as streptomycin, dihydrostreptomycin, and sisomicin and phospholipids such as phosphatidylinositol, phosphatidylcholine, phosphatidylethanolamine, phosphatidylserine and phosphatidylglycerol alone or in combination with other lipids. This invention includes the methods of making aminoglycoside phosphate associated liposomes as described below and particularly the liposomes substantially associating with available aminoalvcoside phosphate.

Further included in this invention is the method of therapeutic treatment of animals including humans with therapeutically effective amounts of aminoglycoside phosphate, liposomally asociated aminoglycoside phosphate, and said phosphate in association with suitable pharmaceutical carrier. Further included is the preparation of the phosphates of aminoglycosides, and particularly the phosphates of streptomycin, dihydrostreptomycin, and sisomicin. Additionally included in this invention is aminoglycoside phosphate and liposomally associated aminoglycoside in the treatment of gram-negative pneumonia.

The liposomes of this invention include liposomes and especially SPLV liposomes comprising at least one lipid and at least one aminoglycoside, preferably in the form of a sulfate salt. Further included are multilamellar vesicle-type liposomes associated with aminoglycosides and phospholipids such as phosphatidylinositol, phosphatidylcholine, phosphatidylethanolamine, phosphatidylserine and phosphatidylglycerol. This invention includes the methods of making aminoglycoside associated liposomes and particularly aminoglycoside sulfate associated liposomes as described below, and particularly the liposomes substantially associating with available aminoglycoside sulfate.

Additionally included are guanadino aminoglycoside liposomes of drug to lipid ratios of greater than about 3:5 (actual weight/weight of lipid).

Further included in this invention is the method of treating an animal including a human with therapeutic doses of aminoglycoside liposomes, and further in association with a suitable pharmaceutical carrier. Particularly noted is the treatment of gram-negative pneumonia.

This invention includes liposomes comprising at least one amphipathic lipid. This invention includes the process of enhancing aminoglycoside to lipid drug ratios in liposomes of the SPLV process by variously drying the lipid-organic solvent-aminoglycoside-aqueous phase mixture to powder, permitting the rehydrated powder to stabilize by standing at about 4°C for at least about 8 hours and preferably for about one day. This invention further includes having dried the lipid-organic solvent-aminoglycoside-aqueous phase mixture over a three to eight hour period and preferably a five hour period. Alternatively, the lipid-organic solvent-aminoglycoside-aqueous phase may be dryed only to a slurry or paste.

This invention further includes removal of liposomally unassociated aminoglycoside from aminoglycoside liposome preparation by low energy sepratory methods such as dialysis or chromotography being careful to avoid high energy sepratory methods such as centrifugation.

### DETAILED DESCRIPTION OF THE INVENTION

The utility of aminoglycoside containing liposomes is described in connection with the treatment of disease in animals in U.S. Patent No. 4,552,803 to Lenk et al. The surprisingly increased efficiency of association of aminoglycoside phosphate by liposomes makes these preparations particularly effective and efficient.

Liposomes are vesicles comprising closed bilayer membranes containing an entrapped aqueous phase. Liposomes may be any variety of unilamellar vesicles (possessing a single membrane bilayer) or multilamellar vesicles (e.g. onion-like structures characterized by concentric membrane bilayers, each separated from the next by an aqueous layer).

The aminoglycoside phosphate liposomes of this invention are formed by methods well known in the art. The original liposome preparation of Bangham et al. (1965, J. Mol. Biol. 13:238-252) involves suspending phospholipids in an organic solvent which is then evaporated to dryness leaving a phospholipid film on the reaction vessel. Then an appropriate amount of aqueous phase is added, the mixture is allowed to "swell", and the resulting liposomes which consist of multilamellar vesicles (hereinafter referred to as MLVs) are dispersed by mechanical means. The structure of the resulting membrane bilayer is such that the hydrophobic (nonpolar) "tails" of the lipid orient toward the center of the bilayer while the hydrophilic (polar) "heads" orient towards the aqueous phase. This technique provides the basis for the development of the small sonicated unilamellar vesicles (hereinafter referred to as SUVs) described by Papahadjapoulos and Miller (1967, Biochim. Biophys. Acta. 135:624-638) and large unilamellar vesicles (hereinafter referred to as LUVs). The teachings of Bangham and those of Papahadjapoulos are herein incorporated by reference.

In the practice of this invention as to aminoglycoside phosphates, a class of liposomes characterized as having substantially equal interlamellar solute distribution is preferred. This preferred class of liposomes is denominated as stable plurilamellar vesicles (SPLV) as defined in U.S. Patent No. 4,522,803 to Lenk et. al. and includes monophasic vesicles as described in U.S. Patent No. 4,588,578 to Fountain et. al. and frozen and thawed multilamellar vesicles (FATMLV) as described in "Solute Distributions and Trapping Efficiencies Observed in Freeze-Thawed Multilamellar Vesicles," Mayer et. al., Biochima et Biophysica Acta. 817: 193-196 (1985).

Large unilamellar vesicles may be modified using an extrusion apparatus by a method described in WO 86/00238. To make LUVET vesicles by this technique, MLVs are extruded under pressures of up to about 4826 kPa (700 psi) through a membrane filter. These vesicles may be exposed to at least one freeze and thaw cycle prior to the extrusion technique; this procedure is described in WO 87/00043.

Another technique that is used to prepare vesicles is one which forms reverse-phase evaporation vesicles (REV), Papahadjopoulos et al., U.S. Patent No. 4,235,871.

The term lipid as used herein shall mean any suitable material resulting in a bilayer such that a hydrophobic portion of the lipid material orients toward the bilayer while a hydrophilic portion orients toward the aqueous phase.

Two general classes of lipid compounds are useful in the present invention as to aminoglycoside phosphates . The most prominent members are highly hydrophobic compounds, such as triglycerides. Corn oil serves as a convenient and economical source of mixed triglycerides, but other vegetable oils, including but not limited to palm kernel oil, coconut oil, soybean oil, sunflower oil, safflower oil, cocoa butter, and the like may be used. Specific molecular species might be employed as well. Such species may include, but are not limited to, trilaurin, trimyristin, tripalmitin and tristearin, or other glyceryl esters in which the fatty acyl chains of these compounds as well as other fatty acids are incorporated in a non-homogeneous fashion. Other broad classes of long chain hydrophobic compounds such as the wide range of cholesterol esters may be used. It has even been found that long chain organic mixtures such as petroleum jelly are acceptable lipid materials.

Further a variety of cholesterols and other sterols and their water soluble derivatives have been used to form aminoglcoside phosphate liposomes; see specifically WO 86/05977. Mayhew et al., WO 85/00968, published March 14, 1985, describes a method for reducing the toxicity of drugs by encapsulating them in liposomes comprising alpha-tocopherol and certain derivatives thereof. Also, a variety of tocopherols and their water soluble derivatives have been used to form liposomes, see CA-A-1 333 049. Preferred of this group are cholesterol hemisuccinate and tocopherol hemisuccinate. The only constraint appears to be that the hydrophobic compounds selected should, when uncomplexed with the other components of this invention, be soluble in a particular organic solvent chosen for use in the manufacture of the liposomes.

The second broad class of lipid materials used in this invention as to aminoglycoside phosphate are amphipathic in character. Hydrophilic character could be imparted to the molecule through the presence of phosphato, carboxylic, sulphato, amino, sulfhydryl, nitro, and other like groups. Hydrophobicity could be conferred by the inclusion of groups that include, but are not limited to, long chain saturated and unsaturated aliphatic hydrocarbon groups and such groups substituted by one or more aromatic, cycloaliphatic or heterocyclic group. The preferred amphipathic compounds are phosphoglycerides, representative examples of which include phosphatidylcholine, phosphatidylethanolamine, lysophosphatidylcholine, lysophosphatidylethanolamine, phosphatidylserine, phosphatidylinositol, phosphatidic acid, dimyristoylphosphatidylglycerol and diphosphatidylglycerol alone or in combination with other lipids. Synthetic saturated compounds such as dimyristoylphosphatidylcholine, dipalmitoylphosphatidylcholine, or distearoylphosphatidylcholine or unsaturated species such as dioleoylphosphatidylcholine or dilinoleoylphosphatidylcholine might also be usable. Other compounds lacking phosphorous, such as members of the sphingolipid and glycosphingolipid families, are also within the group designated as lipid.

Amphipathic lipids are necessary as the primary liposomal structural element for aminoglycosides made by the modified SPLV preparation described below. In forming modified SPLV preparation aminoglycoside liposomes, these amphipathic lipids may be admixed with other lipids including triglycerides and sterols.

As to phosphate aminoglycosides, a method for preparing the sterol containing liposomes involves adding to an aqueous buffer a salt form of an organic acid derivative of a sterol capable of forming closed bilayers in an amount sufficient to form completely closed bilayers which entrap an aqueous compartment. A suspension of multilamellar vesicles is formed by shaking the mixture. The formation of vesicles is facilitated if the aqueous buffer also contains the counterion of the salt in solution.

The application of energy to the suspension, e.g., sonication, or extrusion of the vesicles through a French pressure cell (French Press) or through a porous filter of the appropriate pore size, will convert the multilamellar sterol vesicles to unilamellar vesicles.

Liposomes entrap an aqueous medium which is enclosed by the lipid bilayers. The aqueous medium can be for example, water or water containing a dissolved salt or buffer. Examples of such salts or buffers can be sodium chloride and phosphate buffered saline (PBS). Other buffers include but are not limited to borate, citrate, Tris-HCl(Tris-(hydroxymethyl)-aminomethane hydrochloride), and HEPES (N-2-hydroxyethyl piperazine-N¹-2-ethane sulfonic acid). Buffers may be in the pH range of between about 2.0 and about 14.0. In the preferred embodiment as to aminoglycoside phosphate, the preparations are hydrated with HEPES buffer (150 mM NaCl, 20mM HEPES), pH 7.0, borate buffer (100 mM Na₂HCO₃, 50 mM H₃BO₃, pH 8.5, or citrate buffer (150 mM Na-citrate), pH 8.5.

In a liposome-drug delivery system, the therapeutic agent, here aminoglycoside, is entrapped in the liposome and then administered to the patient to be treated. For example, see Rahman et al., U.S. Patent No. 3,993,754; Sears, U.S. Patent No. 4,145,410; Papahadjopoulos et al., U.S. Patent No. 4,235,871; Schneider, U.S. Patent No. 4,224,179, Lenk, et al., U.S. Patent No. 4,522,803, and Fountain et al., U.S. Patent No. 4,588,578.

Pharmaceutical liposomal preparations are, in the preferred embodiment, delivered in physiological saline or water buffered with phosphate or citrate appropriate for injection.

Optionally, the aminoglycoside phosphate liposomes can be dehydrated, thereby enabling storage for extended periods of time until use. Standard freeze-drying equipment or equivalent apparatus may be used to dehydrate the liposomes. Liposomes may also be dehydrated simply by placing them under reduced pressure. Alternatively, the liposomes and their surrounding medium can be frozen in liquid nitrogen prior to dehydration. Dehydration with prior freezing may be performed in the presence of one or more protective sugars in the preparation, according to the process of Janoff et al., WO 86/01103. Examples of protective sugars that may be used include, but are not limited to, trehalose, maltose, sucrose, glucose, lactose and dextran. Alternatively, multilamellar vesicles may be dehydrated with prior freezing without protective sugars. When the dehydrated liposomes are to be used, rehydration is accomplished by methods which include simply adding an aqueous solution, e.g., distilled water, to the liposomes and allowing them to rehydrate.

The aminoglycosides of this invention are administered associated with liposomes, and if desired, in admixture with a pharmaceutically-acceptable carrier (such as physiological saline or phosphate buffer ) selected with regard to the intended route of administration and standard pharmaceutical practice. Dosages for aminoglycosides when associated with liposomes will often be about that of the aminoglycoside alone; dosages will be set by the prescribing medical professional considering many factors including the age, weight and condition of the patient. The ratio of active ingredient to carrier will naturally depend on the chemical nature, solubility and stability of the aminoglycoside, as well as the dosage contemplated. For parenteral administration or injection via such routes as intravenous, intraperitoneal, intramuscular, subcutaneous, or intra-mammary route, sterile solutions of the liposome composition are prepared. For intravenous use, the total concentration of solutes should be controlled to render the preparation isotonic.

In another example of their use, liposomal associated aminoglycosides may be incorporated into a broad range of topical dosage forms including but not limited to gels, oils, emulsions and the like. For instance, the suspension containing the liposomal associated aminoglycoside may be added to the aqueous phase as an ingredient in the liposome preparation. Such preparations may be administered as topical creams, pastes, ointments, gels, lotions and the like for direct application.

### 1. Phosphate salts of aminoglycosides.

The aminoglycosides each contain one or more amino sugars linked by glycosidic linkages to a basic six-membered carbon ring. Various phosphate salts may be formed by titration with acids having phosphate groups. In general it is easier to form a phosphate salt of an aminoglycoside than link a phosphate to an aminoglycoside covalently.

Depending on a number of factors including the choice of titrant, specific aminoglycoside, solvent and temperature, a number of phosphate salts are theoretically possible.

Clearly, aminoglycosides, having the ability to associate with a number of phosphate moieties, may be utilized in degrees of phosphate association. However, in the practice of this invention, the preferred aminoglycosides will have a ratio of from about 1:1.6 to about a ratio of 1:5 molecules of aminoglycoside to phosphate. As used herein aminoglycoside phosphate will refer to an aminoglycoside associated with at least one phosphate.

### 2. Preparation of Phosphate Form

In general aminoglycoside phosphate may be prepared with any solvent that adequately solubilizes both aminoglycoside and phosphate but is not appreciably acidic. Aqueous solvents, particularly water, are preferred. The pH present will be characteristic of the source of phosphate titrant and the aminoglycoside. Sources of phosphate are phosphoric acids and metal phosphate salts such as sodium or potassium phosphate. The temperature and pressure are not critical and standard temperature and pressure are often most convenient. The temperature should not exceed a temperature at which the aminoglycoside remains stable.

### 3. Preparation of Aminoglycoside Phosphate Liposomes

Liposomes may be prepared by any of a number of the methods disclosed in the above incorporated references. Alternatively a method of making liposomes by the process of mixing an aqueous phase with lipid free of organic solvent may be employed. Monophasic vesicles (MPVs) as described in U.S. Patent No. 4,588,578 are formed by the general method of (a) forming a dispersion of lipid in an organic solvent, (b) combining the dispersion with an aminoglycoside phosphate in an aqueous phase to form a biphasic mixture in which the aqueous phase can be encapsulated, and (c) removing the organic solvent.

More specifically, a lipid or a mixture of lipids and an aqueous component are added to an organic solvent or a combination of organic solvents in amounts sufficient to form a monophase. The solvent or solvents are evaporated until a film forms. Then an appropriate amount of aqueous component is added, and the film is resuspended and agitated in order to form the MPVs.

The organic solvent or combination of solvents used in the process must be miscible with water and once mixed with water should solubilize the lipids used to make the MPVs.

For example, an organic solvent or mixture of solvents which satisfies the following criteria may be used in the process: (1) 5 ml of the organic solvent forms a monophase with 0.2 ml of aqueous component and (2) the lipid or mixture of lipids is soluble in the monophase.

Solvents which may be used in the process of the present invention include but are not limited to ethanol, acetone, 2-propanol, methanol, tetrahydrofuran, glyme, dioxane, pyridine, diglyme, 1-methyl-2-pyrrolidone, butanol-2, butanol-1, isoamyl alcohol, isopropanol, 2-methoxyethanol, or a combination of chlorform and methanol (e.g., in a 1:1 ratio v/v).

The evaporation of solvent should be accomplished at suitable temperatures and pressures which maintain the monophase and facilitate the evaporation of the solvents. In fact, the temperatures and pressures chosen are not dependent upon the phase-transition temperature of the lipid used to form the MPVs. The advantage of this latter point is that heat labile aminoglycosides which have desirable properties can be incorporated in MPVs prepared from phospholipids such as distearoylphosphatidylcholine, which can be formed into conventional liposomes only at temperatures above the phase-transition temperature of the phospholipids.

Stable plurilamellar vesicles of aminoglycoside phosphate, SPLVs, are prepared as follows: An amphipathic lipid or mixture of lipids is dissolved in an organic solvent. Many organic solvents are suitable, but diethyl ether, fluorinated hydrocarbons and mixtures of fluorinated hydrocarbons and ether are preferred. To this solution are added an aqueous phase and the aminoglycoside to be entrapped. This biphasic mixture is converted to SPLVs by emulsifying the aqueous material within the solvent while evaporating the solvent. Evaporation can be accomplished during sonication by any evaporative technique, e.g., evaporation by passing a stream of inert gas over the mixture, by heating, or by vacuum. The volume of solvent used must exceed the aqueous volume by a sufficient amount so that the aqueous material can be completely emulsified in the mixture. In practice, a minimum of roughly 3 volumes of solvent to 1 volume of aqueous phase may be used. In fact the ratio of solvent to aqueous phase can vary to up to 100 or more volumes of solvent to 1 volume aqueous phase. The amount of lipid must be sufficient so as to exceed that amount needed to coat the emulsion droplets (about 40 mg of lipid per ml of aqueous phase). The upper boundary is limited only by the practicality and efficiency, as for example, SPLVs can be made with 15 gm of lipid per ml of aqueous phase.

The process produces liposomes with different supermolecular organization than conventional liposomes. According to the present invention, the entire process can be performed at a temperature range of about 4° - 60°C. regardless of the phase transition temperature of the lipid used. The advantage of this latter point is that heat labile aminoglycosides which have desirable properties can be incorporated in SPLVs prepared from phospholipid such as distearoylphosphatidylcholine, but can be formed into conventional liposomes only at temperatures above their phase-transition temperature.

To form FATMLVs one example of a suitable process is as follows: one or more selected lipids are deposited on the inside walls of a suitable vessel by dissolving the lipids in an organic solvent such as chloroform and then evaporating the organic solvent, adding an aqueous phase containing aminoglycoside phosphate which is to be encapsulated to the vessel, allowing the aqueous phase to hydrate the lipid, and mechanically agitating (for example, by swirling or vortexing) the resulting lipid suspension to produce the liposomes which are then subjected to a freeze-thaw process.

Alternatively, one or more selected lipids can be dispersed by employing mechanical agitation in an aqueous phase to produce multilamellar vesicles (MLVs) which also may be subjected to the freeze-thaw process. The process requires about 1-10 minutes at a temperature above the gel/liquid crystalline transition temperature.

The lipid concentration for producing MLVs is at least about 50 mg/ml aqueous solvent. At lower concentrations, multilamellar vesicles having a high trapping efficiency are more difficult to form. A preferred lipid concentration is between about 100 and 1000 mg/ml aqueous solvent, more preferably 100-600 mg/ml, and still more preferably 100-400 mg/ml. Neither detergent nor organic solvent is required.

The freeze-thaw cycle that results in FATMLVs requires rapid freezing of the dispersed liposome mixture and then warming the frozen mixture in a constant temperature bath, to a temperature which will cause the aqueous phase to melt. The temperature employed is generally above the transition temperature for the gel/liquid crystalline transition. A constant temperature bath of about 25-50°C, preferably about 40°C, is generally effective.

Liquid nitrogen baths have been found to be particularly effective for the freezing step. The number of freeze-thaw cycles affects the properties of the resulting FATMLV. Generally, three or more preferrably about five or more freeze-thaw cycles are required to obtain an equilibrium interlamellar osmotic balance. About five freeze-thaw cycles in liquid nitrogen and a 40°C constant temperature bath, result in preferred FATMLV's.

### 4. Pharmacological Use of Aminoglycosides

The aminoglycosides may be classified as broad spectrum antibiotics. With respect to antibacterial spectrum, there are similarities among them, but there are also considerable differences, hence generalizations should be avoided. The aminoglycosides of this invention and liposomally associated aminoglycosides are useful in therapeutically-effective doses in the treatment of gram-negative pneumonia.

Aminoglycosides may be administered in combination with one or more pharmaceutically acceptable carriers. Such carriers are well known in the art. Aminoglycosides are preferably administered intramuscularly or intravenously. Ophthalmic solutions and ointments are also available for topical ophthalmic applications. Creams are available in some cases for topical application.

However, many considerations are involved in determing an actual dosage including incidence of renal failure, the aminoglycoside in use, the animal and its presenting condition. A therapeutically effective dose of an aminoglycoside will be that dosage which, in view of the specifics of the application, produce the desired result. IM or IV preparations of aminoglycoside phosphate associated liposomes are preferably administered suspended in a saline solution.

### 5. Modified SPLV Preparation of Aminoglycoside Enhanced Drug to Lipid Ratio Liposomes

SPLVs produced by a novel method are prepared as follows: An amphipathic lipid or mixture of lipids is dissolved in an organic solvent. This constitutes the first mixture. Many organic solvents are suitable, but diethyl ether, halogenated hydrocarbons and mixtures of halogenated hydrocarbons and ether are preferred with methylene chloride most preferred. To this solution are added an aqueous phase and the aminoglycoside to be liposomally associated. Aminoglycoside sulfate is most preferred at this stage. This biphasic mixture is converted to SPLVs by emulsifying the aqueous material within the organic solvent while evaporating the solvent and in the preferred emobidment evaporating to dryness. Evaporation can be accomplished by any evaporative technique, e.g., evaporation by passing a stream of inert gas over the mixture, by heating, or by vacuum. Drying, and particularly drying to powder, in the preferred embodiment is accomplished over about 3 to 8 hours and preferably over about 5 hours and preferably while stirring and most preferably stirring at high speed. Drying temperature is between about 25°C to 45°C. The temperature must not be at or above the boiling point of the solvent in use which of course will vary with the pressure of the system. With methylene chloride about 40°C is preferred. If not drying to powder, than drying to a paste or slurry is acceptable. The volume of organic solvent used must be proportionate to the aqueous volume so that the aqueous material can be completely emulsified in the mixture. In practice, a minimum of roughly 1 volume of solvent to 1 volume of aqueous phase may be used. In fact the ratio of solvent to aqueous phase can vary to up to 100 or more volumes of solvent to 1 volume aqueous phase. The amount of lipid must be sufficient so as to exceed that amount needed to coat the emulsion droplets (about 40 mg of lipid per ml of aqueous phase). The upper boundary is limited only by the practicality and efficiency, but SPLVs can be made with 15 gm of lipid per ml of aqueous phase.

If a powder has been formed, the resulting preparation is then rehydrated. It is a limitation of this process that the material be permitted to "stabilize" for a period of time. The temperature for stabilizing is not critical but colder temperatures (above freezing) enjoy less liposomal degradation. Thus stabilizing as performed preferably at about 4°c. The period of stabilization is at minimum about 8 hours and preferably at least about one day. After stabilizing unassociated aminoglycoside may be removed, which is done in the preferred embodiment.

The removal of unassociated aminoglycoside must be done in a manner that does not adversely affect liposomal integerity. Dialysing against a saline solution is such a low energy type non-adverse process. Centrifugation is a high energy process that could adversely affect liposomal integrity and should not be utilized. Other low energy type processes known in the art such as chromatography may also be used.

Prior to the removal of unassociated aminoglycoside, the liposomes may be sized. If for intravenous use in humans these are preferably sized to about 3 to 5 um - the size beyond which capillary blockage can occur.

Sizing can be performed by any method including homogenization and extrusion such as by steel mesh, straight path or tortuous path filtration, including membrane filters of polycarbonate and other polymeric substances.

Optimal results require that the liposome mixture be dried to powder prior to rehydration. A further requirement for optimal results is that the liposomes stand to stabilize overnight prior to removal of unassociated aminoglycoside. Further the most preferred results by this process arise from utilzing the sulfate salt form of aminoglycoside. However, aminoglycoside salts including phosphate, chloride, and tartrate are contemplated as well as aminoglycoside free base. Aminoglycoside salts formed with hydrophobic moieties are also contemplated within this invention. Such hydrophobic moieties are fatty acids, for example, palmitate, myristate, and stearate.

By the foregoing method of preparing liposomes of this invention the aminoglycoside is associated with liposomes at enhanced levels does not remain free in solution. By substantially associated it is to be understood that no more than about 60% of the nonguanadino aminoglycoside present in a preparation is not associated with the liposomes.

Aminoglycoside associated liposomes and particularly nonphosphate aminoglycosides of this invention made by the modified SPLV preparation have enhanced drug to lipid ratios compared to previous techniques. This preparation results in the enhancement of liposomal association for guanadino aminoglycoside liposomes prepared by the process of this invention, such guanadino aminoglycoside liposomes have yielded drug to lipid ratios of greater than about 9:25 (equivalent weight/weight) with over about 60 mg of streptomycin sulfate (act. wt.) associating with 100 mg EPC.

### Example 1 (This example is not part of the claimed invention)

### Preparation of Aminoglycoside Phosphate Liposomes

### Preparation of Precursor Liposomes at Various Gentamicin

Concentrations. 16 roundbottom flasks were set up in groups of four. Each group contained four flasks containing 50, 100, 200, and 300 mg of lipid such as egg phosphatidylcholine (EPC), either as a thin film or in powdered form. The vesicles in Group 1 were made with 50 mg/ml aminoglycoside (in all groups). First, 1.0 ml aliquots of 50 mg/ml aminoglycoside were pipetted into the four separate flasks that made up this group and they were vortexed vigorously. Complete mixing yielded MLV preparations that were homogeneous and had a milky consistency. These samples were transferred to plastic cryovials. The samples then underwent the freeze-thaw process. This process was repeated for the three remaining groups.

The samples in Group 2 were made with 100 mg/ml of aminoglycoside, and the same four starting weights of EPC. Likewise, the four samples in Group 3 were made with 200 mg/ml aminoglycoside, and the four samples in Group 4 were made with 400 mg/ml of aminoglycoside. The resulting set of 16 samples were arranged in groups of four and each contained a different starting proportion of lipid and aminoglycoside.

Freeze-thaw cycle. The aminoglycoside:lipid mixtures were transferred to the cryovials, which were then capped. It was, however, helpful that the cryovial seal allow for the expanding and contracting gasses to vent during freezing and thawing. Each vial was secured on a metal extender used to dip the vial in a liquid nitrogen tank.

Each sample was vortexed vigorously to mix the lipid with the aqueous aminoglycoside. The vial was immediately plunged into a liquid nitrogen container. To enhance drug-lipid interactions, the samples were thoroughly mixed and had a homogeneous, milky consistency upon freezing.

When a sample was completely frozen (approximately one minute), the vial was transferred to a 40°C water bath, and allowed to thaw completely. After thawing, the vials were vortexed vigorously and then immediately started in the next freeze-thaw cycle by plunging the vial back into the liquid nitrogen before the phases had a chance to separate.

A minimum of about five freeze-thaw cycles was required for best results; however, the entrapment of certain compounds has been shown to increase by increasing the number of freeze-thaw cycles to about ten.

### Example 2 (This example is not part of the claimed invention)

### Modified SPLV Preparation of Aminoglycoside Enhanced Drug to Lipid Ratio Liposomes

### Preparation of Precursor Liposomes

One g of lipid (egg phosphatidyl choline "EPC") was dried to a film in 500 ml round bottom flask. The lipid was resuspended using about 50 ml of methylene chloride.

An aqueous solution of aminoglycoside was added to the mixture. The aqueous solution was 0.5 gm gentamicin sulfate (act. wt.) in 9 ml of 0.9% saline (weight/volume). The resulting mixture was agitated by stirring. This process created precursor liposomes.

### Drying, Rehydration, and Standing Process

The liposomal mixture was stirred under nitrogen atmosphere. The pressure was reduced but maintained below boiling until the sample was dried to a powder. This process took about 4 to 5 hours at 40°C. The mixture was rehydrated with about 9 ml of distilled water and 41 ml of 0.9% saline (weight/volume) under nitrogen. The mixture was stirred until a milky color was achieved with no aggregates or clumps of material. This procedure took 2 hours at about 40°C. The mixture was left standing or stabilizing at 4°C for one day.

### Dialyzing

The sample was then sized to about 3um by extrusion at pressures of up to about 700 psi through a polycarbonate straight path membrane filter. After filtration the material was dialyzed about 2 days against 0.9% saline (weight/volume). The dialysis removed substantially all aminoglycoside not liposomally associated.

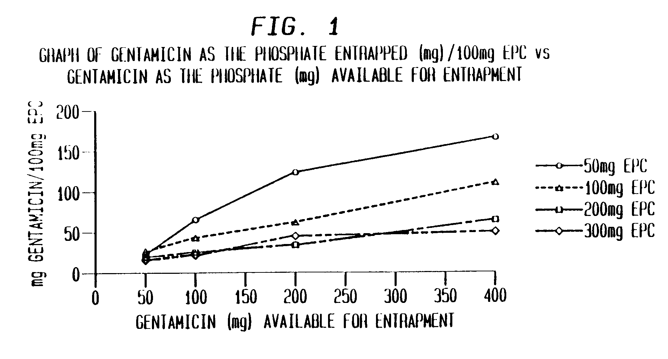

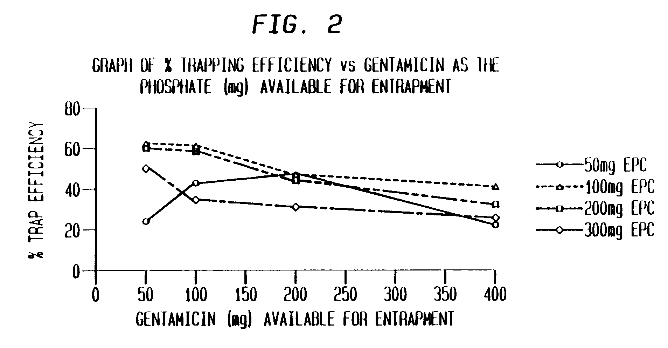

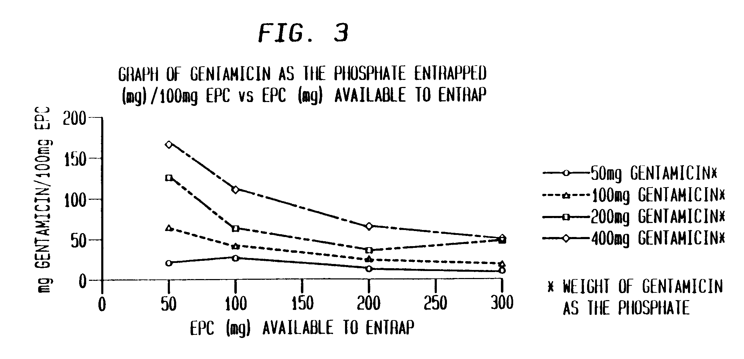

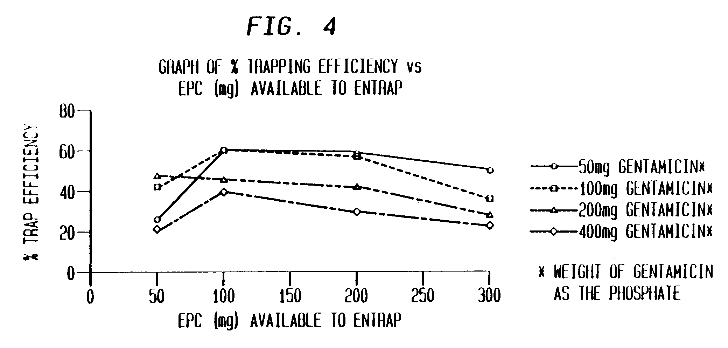

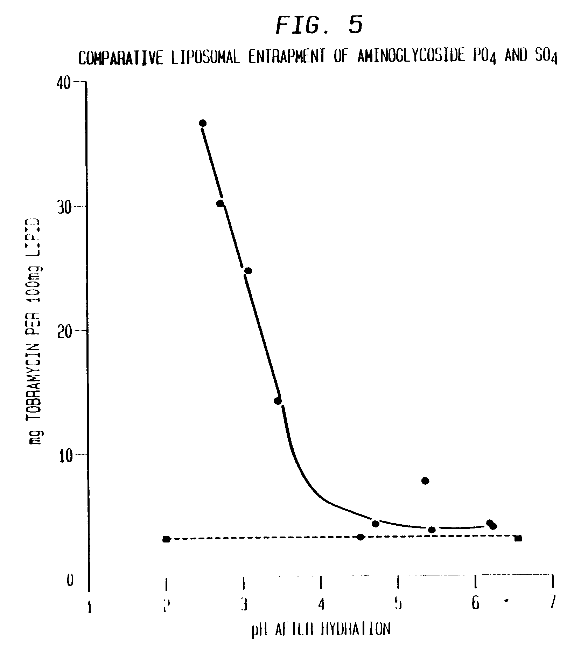

## Claims

1. Liposomes comprising at least one guanidino aminoglycoside present in a drug (eq. wt.) to lipid ratio of a least 9:25 (w/w).

2. The liposomes of claim 1 wherein the aminoglycoside is streptomycin sulfate.

3. The liposomes of claim 1 or 2 wherein the liposomes are multilamellar.

4. The liposomes of claim 3 wherein the multilamellar liposomes are SPLV liposomes.

5. A method of preparing the SPLV liposomes of claim 4, wherein the liposomes comprise at least one guanidino aminoglycoside present in a drug:lipid ratio (eq. wt.) of at least 9:25 (w/w) and wherein the method comprises the steps of:
a) dissolving at least one amphipathic lipid in organic solvent ;
b) adding to the solution formed by step a) an aqueous phase and an aminoglycoside so as to form a biphasic mixture ;
c) emulsifying the aqueous phase of the mixture of step b) within the organic phase while evaporating the organic phase; and
d) drying the material resulting from step c).

6. The method of claim 5, wherein the drying is to a powder, wherein the powder is hydrated and wherein the hydrated material is maintained in the hydrated state for at least about 8 hours ˝.

## Patentansprüche

1. Liposomen, die mindestens ein Guanidinoaminoglykosid umfassen, das in einem Wirkstoff (Equivalentgewicht)- zu -Lipidverhältnis von mindestens 9 : 25 (m/m) vorhanden ist.

2. Liposomen gemäss Anspruch 1, wobei das Aminoglykosid Streptomycinsulfat ist.

3. Liposomen gemäss Anspruch 1 oder 2, wobei die Liposomen multilamellar sind.

4. Liposomen gemäss Anspruch 3, wobei die multilamellaren Liposomen SPLV-Liposomen sind.

5. Verfahren zur Herstellung der SPLV-Liposomen gemäss Ansprüch 4, wobei die Liposomen mindestens ein Guanidinoaminoglykosid umfassen, das in einem Wirkstoff : Lipidverhältnis (Equivalentgewicht) von mindestens 9 : 25 (m/m) vorhanden ist, und wobei das Verfahren die Stufen umfaßt:
a) Lösen mindestens eines amphipatischen Lipids in organischem Lösungsmittel;
b) Zugabe einer wäßrigen Phase und eines Aminoglykosids zur in Stufe a) gebildeten Lösung zur Bildung einer biphasischen Mischung;
c) Emulgieren der wäßrigen Phase der Mischung aus Stufe b) in der organischen Phase, während die organische Phase verdampft wird; und
d) Trocknen des aus Stufe c) resultierenden Materials.

6. Verfahren aus Anspruch 5, wobei das Trocknen bis zu einem Pulver erfolgt, wobei das Pulver hydratisiert wird und wobei das hydratisierte Material während mindestens etwa 8 Stunden im hydratisierten Zustand gehalten wird.

## Revendications

1. Liposomes comprenant au moins un guanidinoaminoglycoside présent dans un rapport médicament (poids équivalent)/lipide d'au moins 9:25 (en poids/poids).

2. Liposomes selon la revendication 1, dans lesquels l'aminoglycoside est le sulfate de streptomycine.

3. Liposomes selon la revendication 1 ou 2, dans lesquels les liposomes sont multilamellaires.

4. Liposomes selon la revendication 3, dans lesquels les liposomes multilamellaires sont des liposomes SPLV.

5. Procédé de préparation des liposomes SPLV de la revendication 4, dans lequel les liposomes comprennent au moins un guanidinoaminoglycoside présent dans un rapport (poids équivalent) médicament/lipide d'au moins 9:25 (en poids/poids) et dans lequel le procédé comprend les étapes consistant à:
a) dissoudre au moins un lipide amphipathique dans un solvant organique;
b) ajouter à la solution formée par l'étape a) une phase aqueuse et un aminoglycoside de manière à former un mélange à deux phases;
c) émulsionner la phase aqueuse du mélange de l'étape b) au sein de la phase organique tout en évaporant la phase organique; et
d) sécher la matière obtenue dans l'étape c).

6. Procédé selon la revendication 5, dans lequel le séchage est fait jusqu'à l'obtention d'une poudre, dans lequel la poudre est hydratée et dans lequel la matière hydratée est maintenue à l'état hydraté pendant au moins environ 8 heures.
